# EUROPEAN PATENT APPLICATION

(11) **EP 2 253 273 A1**
(43) Date of publication of application: **24.11.2010**
(21) Application number: 09177801.9
(22) Date of filing: 02.12.2009
(51) Int. Cl.: A61B 8/08, G06T 7/00

(54) **Ultrasound diagnostic system and method for displaying organ**

(30) Priority: 18.05.2009 KR 20090043057
(71) Applicant: MEDISON CO., LTD., Gangwon-do 250-875 (KR)
(72) Inventor: Hyun, Dong Gyu, 464-725, Gyeonggi-do (KR); Yoo, Jae Heung, 158-056, Seoul (KR)
(74) Representative: Schmid, Wolfgang

(57) **Abstract**

Disclosed is an ultrasound diagnostic system and method. The ultrasound diagnostic system may receive, from a target object, a response signal corresponding to an ultrasound signal transmitted to the target object, may generate an ultrasound image and at least one organ image by using ultrasound data generated based on the received response signal, and may display the generated ultrasound image and the generated at least one organ image.

## Description

### BACKGROUND

### 1. Field

The present invention relates to an ultrasound diagnostic system and method thereof, and more particularly, to an ultrasound diagnostic system for displaying an organ and method thereof.

### 2. Description of the Related Art

An ultrasound diagnostic system transmits an ultrasound signal toward a predetermined interior part of a body from a surface of the body of a target object, and obtains an image related to a section of a soft tissue or a blood vessel by using information of an ultrasound signal that is passed through a tissue inside of the target object or reflected from the tissue inside of the target object. The ultrasound diagnostic system is small and inexpensive, and also, is able to display the predetermined interior part of the target object in real time and has a high stability since it has no coated wire, and thus, the ultrasound diagnostic system is widely used together with an X-ray diagnostic device, a computerized tomography (CT) scanner, a magnetic resonance image (MRI) device, a nuclear medicine diagnostic device, and the like.

A two-dimensional (2D) or three-dimensional (3D) ultrasound image is obtained by the ultrasound diagnostic system and displayed. Particularly, a 3D ultrasound image has an advantage of visualizing an interior of a body and diagnosing without burdensome processes, such as a surgical operation, thereby, being widely used. However, the 3D ultrasound image has a low spatial resolution, and has difficulty in differentiating main features of organs, such as a blood vessel and the like, and thus, there may be restrictions on the use of the 3D ultrasound image.

### SUMMARY

An aspect of the present invention provides an ultrasound diagnostic system and method for displaying an organ, and the ultrasound diagnostic system and method may generate an ultrasound image and at least one organ image by using the same ultrasound data and simultaneously display the at least one organ, and thus, anatomic information and ultrasound information are simultaneously ascertained.

Another aspect of the present invention also provides an ultrasound diagnostic system and method for displaying an organ, and the ultrasound diagnostic system and method may arrange an ultrasound image and an organ image to have the same geometry information, and thus, a location relationship of anatomic information and ultrasound information is intuitively ascertained.

Another aspect of the present invention also provides an ultrasound diagnostic system and method for displaying an organ, and the ultrasound diagnostic system and method may display an ultrasound image and organ image in different colors, and thus, perspective and contrast are more intuitively ascertained.

According to an aspect of the present invention, there is provided an ultrasound diagnostic system for displaying an organ, including a transceiver to transmit an ultrasound signal to a target object and to receive, from the target object, a response signal corresponding to the transmitted ultrasound signal, a data generating unit to generate ultrasound data with respect to the target object based on the response signal, a general image processing unit to generate an ultrasound image with respect to the target object by using the ultrasound data, an organ image processing unit to generate at least one organ image with respect to at least one organ related to the target object by using the ultrasound data, and a display processing unit to display the ultrasound image and the at least one organ image.

Here, the display processing unit includes an image composition unit to combine the ultrasound image and the at least one organ image to generate a composite image, and a display unit to display the composite image.

Also, the ultrasound image is a three-dimensional (3D) Brightness (B) mode ultrasound image, and the at least one organ image is a 3D organ image.

Also, the image composition unit respectively assigns different weights to the ultrasound image and the at least one organ image, and combines the ultrasound image to which a weight is assigned and at least one image to which a weigh is assigned to generate the composite image.

Also, the ultrasound image and the at least one organ image have the same geometry information. The geometry information may include at least one of orientation information, position information, and scale information.

Also, the display processing unit combines the ultrasound image and the at least one organ image based on the geometry information, and displays the composite image.

The ultrasound image and the at least one organ image are respectively displayed in different colors.

According to an aspect of the present invention, there is provided an ultrasound diagnostic method for displaying an organ, including transmitting an ultrasound signal to a target object, receiving a response signal corresponding to the transmitted ultrasound signal from the target object, generating ultrasound data with respect to the target object based on the response signal, generating an ultrasound image with respect to the target object by using the ultrasound data, generating at least one organ image with respect to at least one organ related to the target object by using the ultrasound data, and displaying the ultrasound image and the at least one organ image.

Additional aspects and/or advantages will be set forth in part in the description which follows and, in part, will be apparent from the description, or may be learned by practice of the embodiments.

### EFFECT

According to an example embodiment of the present invention, an ultrasound image and at least one organ image are generated by using the same ultrasound data, and thus, anatomic information and ultrasound image may be simultaneously ascertained.

Also, according to an example embodiment of the present invention, an ultrasound image and at least one organ image are arranged to have the same geometry information, and thus, a location relationship of anatomic information and ultrasound information may be intuitively ascertained.

Also, according to an example embodiment of the present invention, an ultrasound image and at least one organ image are displayed in different colors, and thus, perspective and contrast may be more intuitively ascertained.

Also, according to an example embodiment of the present invention, an ultrasound image and at least one organ image are distinctively processed to respectively have different pseudo maps and different weights.

Also, according to an example embodiment of the present invention, different weights are respectively assigned to at least one organ image, and thus, an organ that is a target of interest may be emphasized when being displayed.

Also, according to an example embodiment of the present invention, an ultrasound image and at least one organ image are respectively embodied by separate rendering pipelines, and thus, a new organ image may be relatively easily added.

### BRIEF DESCRIPTION OF THE DRAWINGS

The patent or application file contains at least one drawing executed in color. Copies of this patent or patent application publication with color drawing(s) will be provided by the Office upon request and payment of the necessary fee. These and/or other aspects, features, and advantages of the invention will become apparent and more readily appreciated from the following description of exemplary embodiments, taken in conjunction with the accompanying drawings of which:
FIG. 1 is a block diagram illustrating an ultrasound diagnostic system for displaying an
   organ according to an example embodiment of the present invention;
FIG. 2 is a flowchart illustrating an ultrasound diagnostic method for displaying an
   organ according to an example embodiment of the present invention;
FIG. 3 is a diagram illustrating an example of displaying a three-dimensional (3D) ultrasound image together with a 3D organ image;
FIG. 4 is a flowchart illustrating an operation of displaying an ultrasound image and an
   organ image according to an example embodiment of the present invention;
FIG. 5A is a diagram illustrating an example of a composite image of a 3D ultrasound
   image and a 3D organ image;
FIG. 5B is a diagram illustrating examples of composite images generated by respectively assigning different weights to a 3D ultrasound image and a 3D organ image; and
FIG. 6 is a diagram illustrating an example of a composite image of a 3D ultrasound image and two 3D organ images.

### DETAILED DESCRIPTION

Reference will now be made in detail to exemplary embodiments of the present invention, examples of which are illustrated in the accompanying drawings, wherein like reference numerals refer to the like elements throughout. Exemplary embodiments are described below to explain the present invention by referring to the figures.

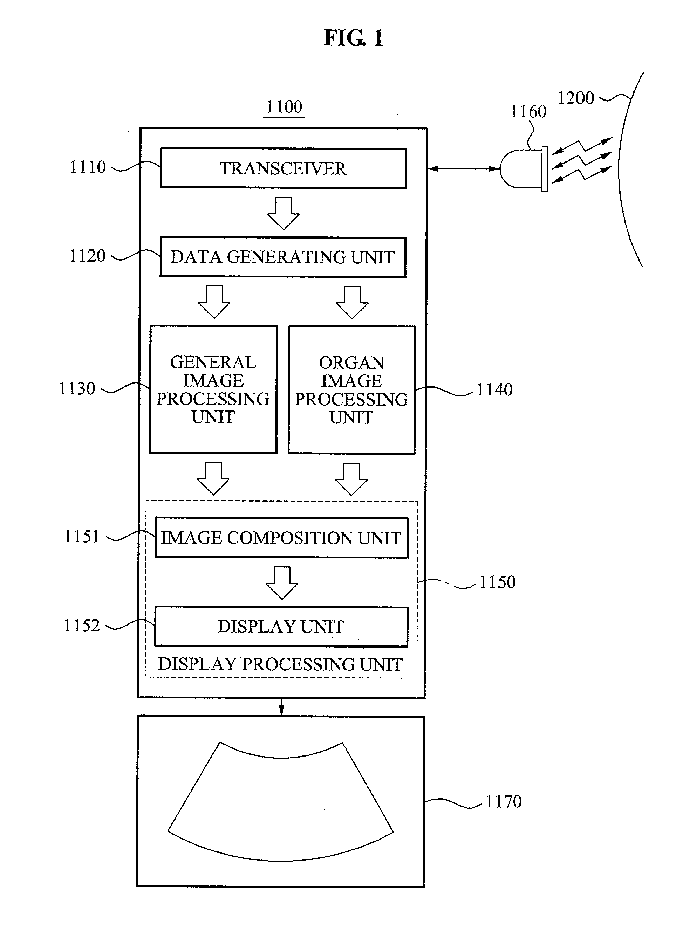

FIG. 1 is a block diagram illustrating an ultrasound diagnostic system 1100 for displaying an organ according to an example embodiment of the present invention.

As illustrated in FIG. 1, the ultrasound diagnostic system 1100 may transmit an ultrasound signal to a target object 1200 through a probe 1160, and may receive a response signal transmitted from the target object 1200. In this instance, a transceiver 1110 may perform data communication with the probe 1160.

Here, the response signal may be at least one of an ultrasound signal passed through the target object 1200 or an ultrasound signal reflected from the target object 1200.

Also, the ultrasound diagnostic system 1100 may generate ultrasound data based on a received response signal, and may generate an ultrasound image with respect to the target object 1200 by using the generated ultrasound data. The generated ultrasound image may be displayed through a display device 1170.

In this instance, the ultrasound diagnostic system 1100 may display a two dimensional (2D) ultrasound image or a three dimensional (3D) ultrasound image through the display device 1170. Also, the ultrasound diagnostic system 1100 may generate an ultrasound image with respect to a region of interest (ROI) included in the target object 1200, and may display the generated ultrasound image with respect to the ROI.

Particularly, the ultrasound diagnostic system 1100 may generate the ultrasound image with respect to the target object 1200 and at least one organ image related to the target object 1200 by using the same ultrasound data, and may display the generated ultrasound image together with the at least one organ image. The at least one organ image may be generated to provide anatomic information with respect to the target object 1200.

As an example, when the ultrasound diagnostic system 1100 generates the ultrasound image with respect to the ROI included in the target object 1200 and the ROI is a liver and surroundings, the at least one organ related to the ROI may be at least one of a blood vessel, a diaphragm, a cyst, a gall bladder, a bile duct, a tumor, and a general tissue. In this instance, the ultrasound diagnostic system 1100 may transmit an ultrasound signal to the liver which is the ROI and the surroundings, and may receive a response signal from the liver and the surroundings to generate an ultrasound image and at least one organ image with respect to the liver which is the target object and surroundings.

Subsequently, the ultrasound diagnostic system 1100 may generate the ultrasound data by using the received response signal. The generated ultrasound data may be used for generating the ultrasound image and the at least one organ image with respect to the ROI.

The ultrasound diagnostic system 1100 may display the generated ultrasound image and the at least one organ image with respect to ROI together, thereby enabling a user to simultaneously ascertain the ultrasound image of the ROI and anatomic information of the ROI.

The ultrasound diagnostic system 1100 includes a transceiver 1110, a data generating unit 1120, a general image processing unit 1130, an organ image processing unit 1140, and a display processing unit 1150. Hereinafter, a function of each element will be described in detail.

The transceiver 1110 may transmit an ultrasound signal to the target object 1200, and may receive a response signal corresponding to the transmitted ultrasound signal, from the target object 1200.

As described above, the response signal may be at least one of an ultrasound signal passed through the target object 1200 or an ultrasound signal reflected from the target object 1200.

The data generating unit 1120 may generate an ultrasound signal with respect to the target object based on the response signal received from the ROI.

The general image processing unit 1130 may generate the ultrasound image with respect to the ROI 1200 by using the generated ultrasound data, and the organ image processing unit 1140 may generate the at least one organ image with respect to the at least one organ related to target object 1200 by using the generated ultrasound data.

The display processing unit 1150 may display the generated ultrasound image and the at least one organ image.

According to an example embodiment of the present invention, the display processing unit 1150 may include an image composition unit 1151 and a display unit 1152.

The image composition unit 1151 may combine the generated ultrasound image and the generated at least one organ image to generate a composite image, and the display unit 1152 may display the generated composite image through the display device 1170.

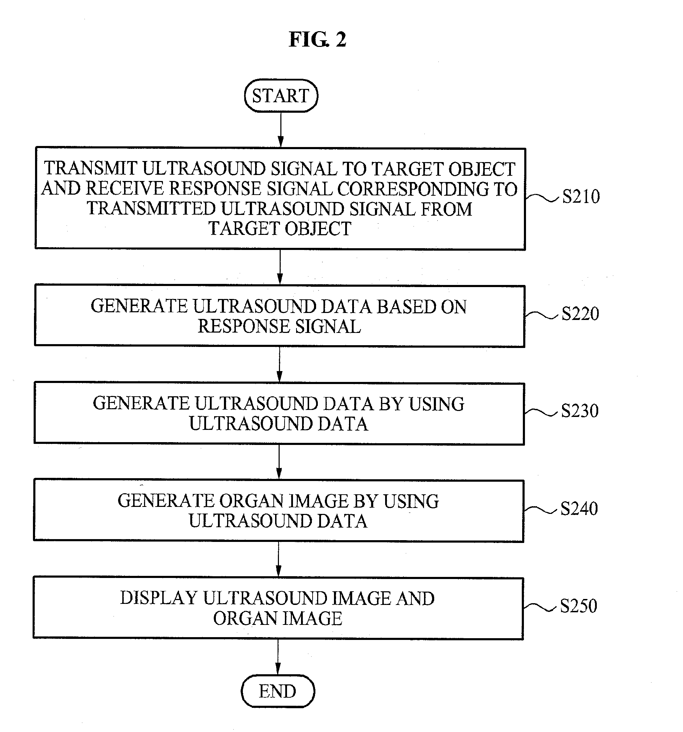

Hereinafter, operations of the ultrasound diagnostic system 1100 according to an example embodiment of the present invention will be described in detail with reference to FIGS. 2 through 6.

FIG. 2 is a flowchart illustrating an ultrasound diagnostic method for displaying an organ according to an example embodiment of the present invention.

The ultrasound diagnostic method for displaying an organ is performed by operations S210 through S250. In this instance, operation S210 is performed by the transceiver 1110, operation S220 is performed by the data generating unit 1120, operation S230 is performed by the general image processing unit 1130, operation S240 is performed by the organ image processing unit 1140, and operation S250 is performed by the display processing unit 1150.

First, in operation S210, the transceiver 1110 transmits an ultrasound signal to the target object 1200, and receives a response signal corresponding to the transmitted ultrasound signal, from the target object 1200.

Particularly, the transceiver 1110 may transmit the ultrasound signal to the target object 1200 through the probe 1160, and may receive an ultrasound signal passed through the target object 1200 or an ultrasound signal reflected from the target object, as the response signal.

In this instance, the probe 1160 may include a plurality of one-dimensional (1D) or 2D transducers. The probe 1160 may generate an ultrasound signal by properly delaying input times of pulses that are respectively inputted to the plurality of transducers, and may transmit the generated ultrasound signal to the target object 1200 according to a transmission scan line.

Also, the response signal, that is, an ultrasound signal passed through the target object 1200 or reflected from the target object 1200, may have different receipt times and may be inputted to the plurality of transducers, and the transceiver 1110 may receive the response signal from the probe 1160.

Also, according to an example embodiment of the present invention, the probe 1160 may be a 3D probe. In this instance, the transceiver 1110 may receive a 3D response signal through the probe 1160.

In operation S220, the data generating unit 1120 may generate ultrasound data based on the response signal.

According to an example embodiment of the present invention, the data generating unit 1120 may generate a plurality of 2D ultrasound data based on the response signal inputted from the transceiver 1110, and may generate 3D ultrasound data from the generated plurality of 2D ultrasound data.

As an example, the data generating unit 1120 may perform an envelope detection to detect a size of the response signal based on the response signal, and may generate the 2D ultrasound data. In this instance, the data generating unit 1120 may generate the 2D ultrasound data based on data obtained from location information of a plurality of points existing in each scan line and data obtained from each point, and the 2D ultrasound data may include coordinates of each point on an X-Y coordinate system, angle information of each scan line with respect to a vertical scan line, data obtained from each point, and the like.

Also, according to another example embodiment of the present invention, the data generating unit 1120 may receive the 3D response signal from the transceiver 1110 and may generate 3D ultrasound data based on the received 3D response signal. In this instance, the probe 1160 may be the 3D probe as described in the above description.

In operation S230, the general image processing unit 1130 may generate an ultrasound image by using the ultrasound data. In this instance, the generated ultrasound image may be a 3D ultrasound image.

As an example, the general image processing unit 1130 may generate a 3D Brightness (B) mode ultrasound image with respect to the target object 1200 by using the ultrasound data. In this instance, the general image processing unit 1130 may generate the 3D B mode ultrasound image by using 3D ultrasound data.

Also, when the ultrasound diagnostic system 1100 generates a 3D B mode ultrasound image with respect to an ROI included in the target object 1200, the ROI and other related organs may be displayed in the 3D B mode ultrasound image. As an example, when the ROI is a liver or the liver and surroundings thereof, a blood vessel connected to the liver, a diaphragm, a cyst, a gall bladder, a bile duct, a tumor, a general tissue, and the like may be displayed in the 3D B mode ultrasound image. However, since the 3D B mode ultrasound image has a low spatial resolution, the liver and the surroundings may not be clearly distinguished.

Accordingly, the organ image processing unit 1140 generates an organ image by using the ultrasound data in operation S240 to simultaneously display an image with respect to other organs related to the ROI and the ultrasound image with respect to the ROI, thereby providing a user with meaningful information with respect to the ROI.

In this instance, the organ image processing unit 1140 may perform separate rendering with respect to at least one organ, and may generate at least one organ image respectively corresponding to the at least one organ. For this, the organ image processing unit 1140 may include at least one rendering unit (not illustrated), and the at least one rendering unit (not illustrated) may perform rendering for each of the at least one organ, and may generate the at least one organ image.

Accordingly, when, after generating the ultrasound image and the at least one organ image, the ultrasound diagnostic system 1100 intends to additionally generate and display an organ image of another organ, the organ image processing unit 1140 may perform a separate rendering process with respect to the desired organ and may generate the organ image of the desired organ. Accordingly, the ultrasound diagnostic system 100 may easily add and display an organ image.

Also, the organ image may be a 3D organ image. Particularly, the organ image processing unit 1140 may generate the 3D organ image by using an ultrasound data that is the same as the ultrasound data used for generating the ultrasound image.

According to an example embodiment of the present invention, the organ image processing unit 1140 may increase an edge contrast of the ultrasound image that is generated by using the ultrasound data, and may generate the 3D organ image.

As an example, when the ROI is the liver or the liver and surroundings thereof, and an organ related to the liver is a diaphragm, the organ image processing unit 1140 may increase the edge contrast of the ultrasound data, and may extract an image of the diaphragm from the ultrasound data having the increased edge contrast to generate a 3D organ image of the diaphragm. In this instance, the organ image processing unit 1140 may perform a Hessian matrix-based flatness test to extract the diaphragm. Particularly, the diaphragm may be expressed as a curved surface in the 3D B mode ultrasound image with respect to the liver, and thus, the organ image processing unit 1140 may extract an area where a change in a pixel intensity in a vertical direction to the surface is greater than a change in a pixel intensity in a horizontal direction to the surface, as the diaphragm.

As another example, when the ROI is the liver or the liver and the surroundings, and the organ related to the liver is a blood vessel, the organ image processing unit 1140 may increase an edge contrast of the ultrasound data, and may extract an image of the blood vessel from the ultrasound data having the increased edge contrast to generate a 3D organ image with respect to the blood vessel. In this instance, the organ image processing unit 1140 may extract the blood vessel through an ROI masking, segmentation, and classification.

Generating of the 3D ultrasound image and the 3D organ image based on the described methods may be merely example embodiments of the present invention, and the present invention includes various example embodiments that generate both a 3D ultrasound image of an ROI and a 3D organ image of an organ related to the ROI from the same ultrasound data.

In operation S250, the display processing unit 1150 may display the generated ultrasound image and the generated organ image.

According to an example embodiment of the present invention, the ultrasound image and the organ image may be displayed in different colors from each other. When there is a plurality of organ images, the plurality of organ images may also be display in different colors from each other.

The image processing unit 1130 and the organ image processing unit 1140 may perform assigning of color to the ROI and the at least one organ, and the display processing unit 1150 may also perform the assigning of color.

As an example, when the general image processing unit 1130 and the organ image processing unit 1140 assign different colors with respect to the ROI and the at least one organ, the general image processing unit 1130 and the organ image processing unit 1140 may respectively generate, based on different color tables, a 3D ultrasound image and at least one 3D organ image which have different colors from each other. When there is a plurality of organs related to the ROI, the organ image processing unit 1140 may generate a plurality of organ images having different colors from each other based on different color tables for each of the plurality of organs. In this instance, the color table may include a pseudo map.

As another example, when the display processing unit 1150 assigns different colors to the ROI and at least one organ, different color tables may be stored in the display processing unit 1150. Also, the different processing unit 1150 may assign different colors, based on the stored color tables, to an ultrasound image and at least one organ image that are inputted from the general image processing unit 1130 and the organ image processing unit 1140, and may display them.

As an example, when the ROI is the liver, and the organ related to the liver is a diaphragm and a blood vessel, the general image processing unit 1130 may use yellow as a color of an ultrasound image with respect to the liver and may generate the ultrasound image, and the organ image processing unit 1140 may use green as a color of an organ image with respect to the diaphragm, may use red as a color of an organ image with respect to the blood vessel, and may generate the organ image.

Through this, the ultrasound diagnostic system 1100 may provide the user with a composite image having a more intuitive perspective and contrast.

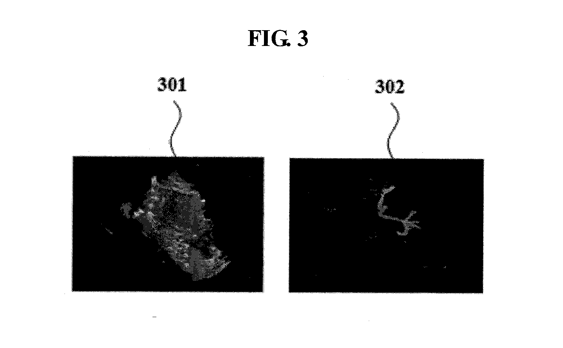

FIG. 3 is a diagram illustrating an example of displaying a 3D ultrasound image 301 together with a 3D organ image 302. As illustrated in FIG. 3, the display processing unit 1150 may display the 3D ultrasound image 301 and the 3D organ image 302 together, both being generated based on the same ultrasound data.

Also, the 3D ultrasound image 301 and the 3D organ image 302 may be displayed in different colors. That is, as illustrated in FIG. 3, the 3D ultrasound image 301 with respect to an ROI may be displayed in yellow and the 3D organ image 302 with respect to an organ, namely, a blood vessel, may be displayed in red.

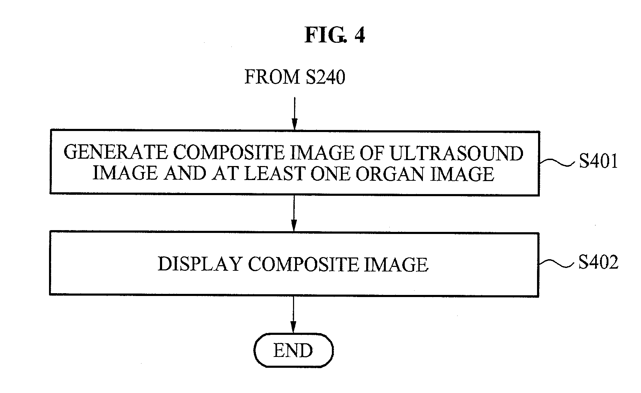

FIG. 4 is a flowchart illustrating operation S250 of displaying an ultrasound image and an organ image according to an example embodiment of the present invention.

As illustrated in FIG. 4, operation S250 may include operation S401 and operation S402. In this instance, operation S401 may be performed in an image composition unit 1151 and operation S402 may be performed in a display unit 1152.

In operation S401, the image composition unit 1151 may generate a composite image by combining an ultrasound image and at least one organ image, and in operation S402, the display unit 1152 may display the composite image generated in the image composition unit 1151.

As an example, in operation S401, the image composition unit 1151 may generate the composite image by overlapping the ultrasound image with the at least one organ image. In this instance the ultrasound image and the at least one organ image may have the same geometry information, and the image composition unit 1151 may overlap the ultrasound image with the at least one organ image based on the geometry information. The geometry information may include at least one of orientation information, position information, and scale information.

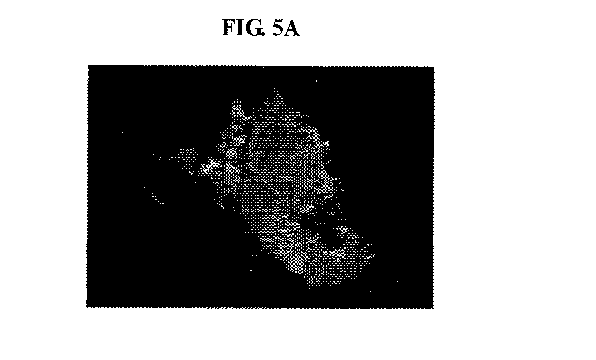

FIG. 5A is a diagram illustrating an example of composite image of a 3D ultrasound image and a 3D organ image. As illustrated in FIG. 5A, the 3D ultrasound image and the 3D organ image may overlap each other and may be displayed.

Also, according to an example embodiment of the present invention, the image composition unit 1151 may respectively assign different weights to an ultrasound image and at least one organ image to emphasize an organ that is a target of interest, and may combine the ultrasound image and the at least one organ image, different weights being assigned to the images, to generate the composite image.

As an example, the image composition unit 1151 may assign a first weight to the ultrasound image and may assign a second weight to the organ image, and may generate the composition image by overlapping the ultrasound image where the first weight is assigned with the organ image where the second weight is assigned.

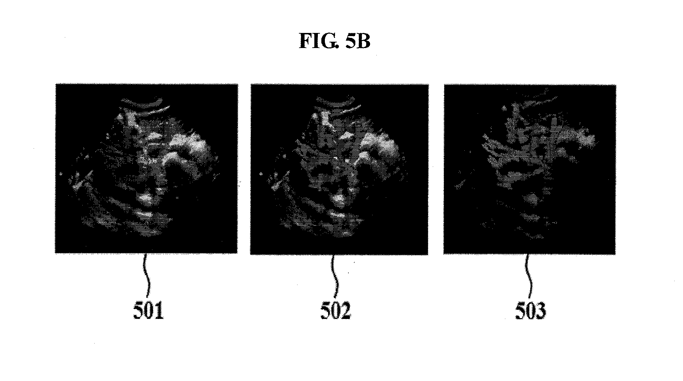

FIG. 5B is a diagram illustrating examples of composite images generated by respectively assigning different weights to a 3D ultrasound image and a 3D organ image.

The composite image 501 is generated by assigning a weigh "1" to a 3D ultrasound image and assigning a weight "0.5" to a 3D organ image, the composite image 502 is generated by assigning the weight "0.5" to the 3D ultrasound image and assigning the weight "1" to the 3D organ image, and the composite image 503 is generated by respectively assigning the weight "1" to the 3D ultrasound image and the 3D organ image.

As illustrated in FIG. 5B, an image where a higher weight is assigned is more clearly displayed. Accordingly, a target of a user's interest may be more clearly displayed.

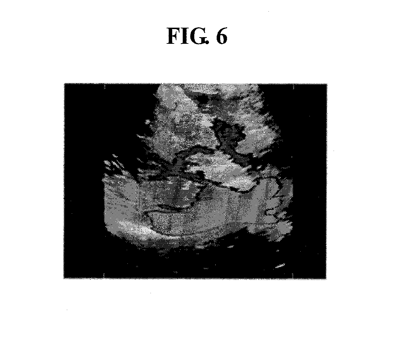

FIG. 6 is a diagram illustrating an example of a composite image of a 3D ultrasound image and two 3D organ images.

As illustrated in FIG. 6, the display unit 1152 may respectively assign different colors to a 3D ultrasound image, a 3D organ image with respect to a first organ (diaphragm), and a 3D organ image with respect to a second organ (blood vessel), and may display the composite image where the 3D ultrasound image, the 3D organ image with respect to the first organ, and the 3D organ image with respect to the second organ overlap each other, different colors being assigned to the above described three images. Here, an ROI is displayed in yellow, the diaphragm is displayed in green, and the blood vessel is displayed in red.

The ultrasound diagnostic method according to the above-described example embodiments may be recorded in computer-readable media including program instructions to implement various operations embodied by a computer. The media may also include, alone or in combination with the program instructions, data files, data structures, and the like. Examples of computer-readable media include magnetic media such as hard disks, floppy disks, and magnetic tape; optical media such as CD ROM disks and DVDs; magneto-optical media such as optical disks; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter. The described hardware devices may be configured to act as one or more software modules in order to perform the operations of the above-described example embodiments, or vice versa.

Although a few exemplary embodiments of the present invention have been shown and described, the present invention is not limited to the described exemplary embodiments. Instead, it would be appreciated by those skilled in the art that changes may be made to these exemplary embodiments without departing from the principles and spirit of the invention, the scope of which is defined by the claims and their equivalents.

## Claims

1. An ultrasound diagnostic system for displaying an organ, comprising:
a transceiver to transmit an ultrasound signal to a target object and to receive,
from the target object, a response signal corresponding to the transmitted ultrasound signal;
a data generating unit to generate ultrasound data with respect to the target object based on the response signal;
a general image processing unit to generate an ultrasound image with respect to the target object by using the ultrasound data;
an organ image processing unit to generate at least one organ image with respect to at least one organ related to the target object by using the ultrasound data; and
a display processing unit to display the ultrasound image and the at least one organ image.

2. The system of claim 1, wherein the display processing unit comprises:
an image composition unit to combine the ultrasound image and the at least one organ image to generate a composite image; and
a display unit to display the composite image.

3. The system of claim 2, wherein the image composition unit respectively assigns different weights to the ultrasound image and the at least one organ image, and combines the ultrasound image to which a weight is assigned and the at least one image to which a weight is assigned to generate the composite image.

4. The system of claim 1, wherein:
the ultrasound image is a three-dimensional (3D) Brightness (B) mode ultrasound image; and
the at least one organ image is a 3D organ image.

5. The system of claim 1, wherein the organ image processing unit increases an edge contrast of the ultrasound image generated by using the ultrasound data and generates the at least one organ image.

6. The system of claim 1, wherein the ultrasound data is 3D ultrasound data.

7. The system of claim 1, wherein:
the target object includes a region of interest (ROI);
the ultrasound image is an ultrasound image with respect to the ROI; and
the at least one organ image is an organ image with respect to at least one organ related to the ROI.

8. The system of claim 7, wherein:
the ROI is a liver; and
the at least one organ includes at least one of a blood vessel, a diaphragm, a tumor, a gall bladder, a bile duct, and a general tissue which are related to the liver.

9. The system of claim 1, wherein the ultrasound image and the at least one organ image have the same geometry information.

10. The system of claim 9, wherein the geometry information includes at least one of orientation information, position information, and scale information.

11. The system of claim 9, wherein the display processing unit combines the ultrasound image and the at least one organ image based on the geometry information, and displays the composite image.

12. The system of claim 1, wherein the ultrasound image and the at least one organ image are respectively displayed in different colors.

13. An ultrasound diagnostic method for displaying an organ, comprising:
transmitting an ultrasound signal to a target object;
receiving a response signal corresponding to the transmitted ultrasound signal from the target object;
generating ultrasound data with respect to the target object based on the response signal;
generating an ultrasound image with respect to the target object by using the ultrasound data;
generating at least one organ image with respect to at least one organ related to the target object by using the ultrasound data; and
displaying the ultrasound image and the at least one organ image.

14. The method of claim 13, wherein the displaying comprises:
combining the ultrasound image and the at least one organ image to generate a composite image; and
displaying the composite image.

15. At least one medium comprising computer readable instructions implementing the method of claim 13.
